# EUROPÄISCHE PATENTSCHRIFT

(11) **EP 2 732 805 B1**
(45) Veröffentlichungstag und Bekanntmachung des Hinweises auf die Patenterteilung: **17.05.2017**
(21) Anmeldenummer: 13187825.8
(22) Anmeldetag: 09.10.2013
(51) Int. Cl.: A61K 8/41, A61K 8/06, A61Q 1/14, A61K 8/03

(54) **Zweiphasen Produkt**
Dual phase product
Produit à deux phases

(30) Priorität: 26.10.2012 DE 102012219641
(43) Veröffentlichungstag der Anmeldung: 21.05.2014
(73) Patentinhaber: Beiersdorf AG, 20253 Hamburg (DE)
(72) Erfinder: Fischer, Britta, 22880 Wedel (DE); Schornstein, Ina, 22529 Hamburg (DE); Hetzel, Frank, 21261 Welle (DE)

(56) Entgegenhaltungen:
- WO-A2-2012/084410
- DE-A1-102006 030 532
- DE-A1-102007 018 225
- DE-A1-102010 013 272
- DE-A1-102010 013 275
- DE-A1-102010 013 277
- US-A1- 2002 068 074

## Beschreibung

Die Erfindung ist ein Zweiphasenprodukt umfassend Benzethoniumchlorid. Benzethoniumchlorid führt zu einer Verlangsamung der Trenngeschwindigkeit der zwei Phasen nach dem Vermischen.

Zwei-Phasen-Zubereitungen werden vor Gebrauch durch Schütteln vermischt. Nach dem Gebrauch entmischen sich die Produkte innerhalb kurzer Zeit. Das Vorliegen zweier getrennter Phasen wird dem Verbraucher etwa durch unterschiedliche Färbung der Phasen angezeigt.

Kosmetische Zweiphasenprodukte sind beispielsweise im Bereich der Haarpflege und - styling oder als Make-up Entferner bekannt. Sie bestehen aus einem bestimmten Anteil einer Öl- und einer Wasserphase.

Im Bereich der Reinigungsprodukte hat sich gezeigt, dass zweiphasige Systeme eine bessere Reinigungsleistung haben, besonders bei wasserfestem Make-up. Hier wird die gut reinigende Wirkung von Ölen mit Wasser kombiniert, welches die ölige Sensorik minimiert.

Um ein zwei Phasenprodukt zu bilden, bedarf es einer Phase mit Wasser und einer Phase mit Öl. Im Ruhezustand sind diese beiden Phasen idealerweise optisch klar voneinander getrennt zu erkennen. Durch die unterschiedlichen Dichten vermischen sich diese beiden Phasen nicht selbstständig miteinander und bilden dadurch ein horizontal getrenntes 2-phasiges System aus. Durch das Einbringen von Energie z.B. durch Schütteln, kann man die Phasen kurzzeitig miteinander vermischen. Aufgrund des Dichteunterschiedes und einer hohen Grenzflächenspannung (GFS) von Öl gegenüber Wasser kommt es allerdings danach wieder zu einer sehr schnellen Auftrennung in die zwei Phasen.

Um 2-phasige Produkte richtig anwenden zu können, sollte die Trenngeschwindigkeit und insbesondere der Trennungsstart vorteilhafterweise verlangsamt werden, da man ansonsten nur eine Phase bzw. zum größten Teil nur eine Phase entnehmen und anwenden kann. D.h. bei umgehender Phasentrennung nach dem Schütteln lässt sich mitunter nur eine Phase entnehmen und man reinigt sich z.B. nur mit Wasser. Dies hat dadurch eine geringere Reinigungsleistung zur Folge, da die reinigende Wirkung des Öls fehlt.

Die auf dem Markt befindlichen Produkte nehmen entweder in Kauf, dass die Trennung zu schnell ist oder sie versuchen das Problem durch den Zusatz an Tensiden oder Emulgatoren zu lösen. Durch deren Zusatz wird die Grenzflächenspannung (GFS) zwar herab gesetzt, aber der Zusatz dieser Rohstoffe kann zu Trübungen/Ausfällungen in der Zubereitung führen. Es kann sich eine sogenannte 3. Phase bilden, die einen visuellen Eindruck eines weißlichen Niederschlags hervorruft, der als Trübung sichtbar wird. Dies benachteiligt sowohl die Optik von klaren Systemen als auch die mikrobiologische Stabilität des Produktes. Der Zusatz an Emulgatoren oder Tensiden kann auch dazu führen, dass sich Teile des Produktes durch das Schütteln dauerhaft emulgieren und so die Öl- und Wasserphase teilweise oder sogar komplett weiß emulgiert bleibt.

US 20020068074 A1 offenbart zweiphasige Zubereitungen umfassend ein Emulsionssystem enthaltend ein kationisches und ein nicht-ionisches Tensid. Als bevorzugtes kationisches Tensid ist Benzalkoniumchlorid zu wählen.

Weiterer Nachteil bestehender Zweiphasenprodukte ist die zu beobachtende Tröpfchenbildung an Behälterwandungen, die als haftende große klare Tropfen auftreten und als "Fettaugen" bezeichnet werden. Weiterhin kann es zu unschönen optischen Problemen, wie Schaumbildung an der Phasengrenze, Bildung einer kleinen 3. Phase (emulgiert) oder auch Hautunverträglichkeiten, besonders im Bereich empfindlicher Haut, wie beispielsweise am Auge, kommen.

Wünschenswert ist es daher Zweiphasenzubereitungen zur Verfügung zu stellen, die die Nachteile des Standes der Technik nicht aufweisen. Insbesondere ist es wünschenswert Zweiphasenzubereitungen bereit zu stellen, die es dem Anwender ermöglichen, beide Phasen ohne Zusatzaufwand zur Anwendung zu bringen. Erstrebenswert ist es darüber hinaus Zweiphasenzubereitungen zur Verfügung zu stellen, die nach dem Vermischen dann wieder eine optisch klare Trennung zeigen.

Erstaunlicherweise konnte dies nicht allein durch Angleichung der Dichten von Öl- und Wasserphase erreicht werden. Es zeigte sich, dass es zusätzlich erforderlich ist, die Grenzflächenspannung herabzusetzen.

Die Erfindung ist eine zweiphasige kosmetische oder dermatologische Zubereitung umfassend eine Öl- und eine Wasserphase und Benzethoniumchlorid.

Benzethoniumchlorid (CAS Nr 121-54-0) ist ein hygroskopischer Feststoff mit antimikrobieller Aktivität, welcher leicht löslich in Wasser ist. Benzethoniumchlorid wird in der Kosmetikindustrie und bei Arzneimitteln als Konservierungsmittel eingesetzt und hat die Struktur

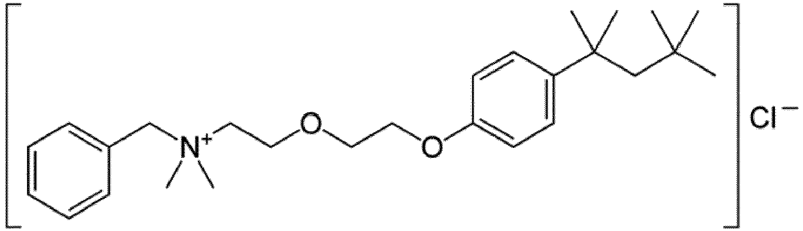

Vorteilhaft wird Benzethoniumchlorid im Bereich von 0,01 bis 1 Gew.%, insbesondere 0,03 bis 0,05 Gew.%, bezogen auf die Gesamtmasse der Zubereitung, eingesetzt.

Entscheidender Unterschied zwischen den erfindungsgemäßen Zubereitungen und Emulsionszubereitungen ist die Phasentrennung.

Unter einer Emulsion versteht man ein fein verteiltes Gemisch zweier normalerweise nicht mischbarer Flüssigkeiten ohne sichtbare Entmischung.

Ein zweiphasige Zubereitung hat aber gerade dieses Merkmal der sichtbaren Entmischung, ansonsten es keine Zweiphasenzubereitung sein kann.

Für den Fachmann der Kosmetik ist damit klar zu unterscheiden zwischen einer Emulsion und einer Zweiphasenzubereitung.

Eine Zweiphasenzubereitung wird allein durch Energieeintrag, wie Schütteln, zu einer Mischung. Nach kürzester Zeit entmischt sich aber eine Zweiphasenzubereitung wieder. Eine kosmetische Emulsion ist jedoch langzeitstabil ohne Entmischung.

Die Ölphase der erfindungsgemäßen Zubereitung umfasst oder wird bevorzugt aus einem oder mehreren Bestandteilen aus der Gruppe Isododecane, Isohexadecane, Isopropylpalmitat, C13-16 Isoparaffin, Coco-Caprylat/Caprat und Dimethicon gewählt.

Vorteilhaft ist die Ölphase nur aus einem oder mehreren der genannten Ölbestandteile gebildet.

In einem Vergleichstest wurden zweiphasige Zubereitungen entsprechend nachfolgender Tabellen untersucht.

**Tabelle 1: Zweiphasige Zubereitungen mit Trübungsbildung (Gew.%)**

| INCI | 18 | 19 | 54 |
|---|---|---|---|
| Isopropyl Palmitate | 8,9000 | 8,9000 | 8,9000 |
| Isododecane | 30,2000 | 30,2000 | 30,2000 |
| Cyclomethicone | 9,9000 | 9,9000 | 9,9000 |
| Cl 61565 gelöst in Isopropyl Palmitate | 0,5000 | 0,5000 | 0,5000 |
| Cl 60725 gelöst in Isopropyl Palmitate | 0,5000 | 0,5000 | 0,5000 |
| | | | |
| Methylisothiazolinone | 0,0400 | 0,0400 | 0,0400 |
| Phenoxyethanol | 0,6000 | 0,6000 | 0,6000 |
| Sodium Chloride | 0,5000 | 0,5000 | 0,5000 |
| Aqua | 47,7800 | 47,8130 | 47,7600 |
| Aqua + Trisodium EDTA | 1,0000 | 1,0000 | 1,0000 |
| Aqua + Decyl Glucoside | 0,0800 | 0,0470 | |
| Poloxamer 184 | | | 0,1000 |

Bei den Zubereitungen 18, 19, 54 wurde bereits bei der Bildung der Wasserphase ein Niederschlag beobachtet. Bei genauem Betrachten der untersuchten Ansätze ist zu erkennen, dass es sich bei dem Niederschlag um eine andere flüssige Phase, nicht aber um einen Feststoff handelt. Durch Schütteln kommt es zudem zu einer feinen Tröpfchenbildung und -verteilung. Bei der abgetrennten Phase handelt es sich nach erfolgter IR- und Raman-Analyse um ein Gemisch aus Wasser, Phenoxyethanol und Decyl Glucoside bzw. aus Wasser, Phenoxyethanol und Poloxamer 184.

**Tabelle 2: Zweiphasige Zubereitungen mit Trübungsbildung im Vergleich zu erfindungsgemäßer Zubereitung (Gew.%)**

| INCI (EU) | 238 | 388 | 389 | 508 |
|---|---|---|---|---|
| Helianthus Annuus Seed Oil + Centaurea Cyanus Flower Extract | | | | 0,0100 |
| Isopropyl Palmitate | 18,8000 | 10,8000 | 13,8000 | 9,5000 |
| Isododecane | 30,2000 | 30,2000 | 30,2000 | 30,4900 |
| Caprylic/Capric Triglyceride | | | 5,0000 | |
| Cl 61565 gelöst in Isopropyl Palmitate | 0,5000 | 0,5000 | 0,5000 | 0,5000 |
| Cl 60725 gelöst in Isopropyl Palmitate | 0,5000 | 0,5000 | 0,5000 | |
| Caprylyl Methicone | | 8,0000 | | |
| Dimethicone | | | | 9,5000 |
| Methylisothiazolinone | 0,0400 | 0,0400 | 0,0400 | |
| Phenoxyethanol | | 0,6000 | 0,6000 | 0,3000 |
| Benzethonium Chloride | | | | 0,0400 |
| Sodium Lauroyl Glutamate | 0,3200 | | | |
| Sodium Chloride | 0,5000 | 0,5000 | 0,5000 | 0,2000 |
| Aqua | 48,1400 | 47,7930 | 47,7930 | 48,8930 |
| Aqua + Trisodium EDTA | 1,0000 | 1,0000 | 1,0000 | 0,5000 |
| Aqua + Caprylyl/Capryl Glucoside | | 0,0670 | 0,0670 | 0,0670 |
| | Trübung Wasserphase durch Tensidzugabe | Trübung Wasserphase durch Tensidzugabe | Trübung Wasserphase durch Tensidzugabe | klare Wasserphase auch nach Tensidzugabe |

Durch den Einsatz von Benzethonium Chlorid konnten nichtionische Tenside wie Caprylyl/Capryl Glucoside, Decyl Glucoside oder auch Polyglyceryl-10 Laurate eingearbeitet werden, ohne das es zu Ausfällungen (optisch als Trübung erkennbar) kommt.

Überraschenderweise konnte durch den Einsatz des Konservierungsmittels Benzethonium Chlorid die Grenzflächenspannung extrem herab gesetzt werden und die Trenngeschwindigkeit von Öl- und Wasserphase dadurch deutlich verlangsamt werden. Ein Niederschlag bei der Zubereitung der Wasserphase, wie in den Beispielen 18, 19, 54, 238, 388, 389 trat ebenfalls nicht auf.

In den Untersuchungen wurde anstelle von Methylisothiazolinone Benzethoniumchlorid eingesetzt. Der Anteil an Phenoxyethanol, Trisodium EDTA und Sodium Chloride konnte verringert werden. Der Zusatz von verschiedenen nichtionischen Tensiden z.B. Caprylyl/Capryl Glucoside, Decyl Glucoside oder auch Polyglyceryl-10 Laurate wurde dadurch möglich. Die Wasserphase blieb klar.

Die Trennung von Wasser- und Ölphase, beispielsweise in einem Verhältnis von 50:50 Gew.%, vorzugsweise aber 50:50 Vol.%, startet ohne Zusatz von Benzethoniumchlorid innerhalb von 1 - 3 Sekunden (je nach Intensität des Energieeintrages und Art des Öles). Nach weniger als 40 Sekunden sind die untersuchten Proben komplett wieder in 2-Phasen getrennt.

Erstaunlicherweise kann bei der erfindungsgemäßen Probe allein durch den Zusatz von Benzethoniumchlorid (z.B. zu 0,04 Gew.%) der Trennungsstart auf 20 - 25 Sekunden verzögert werden.

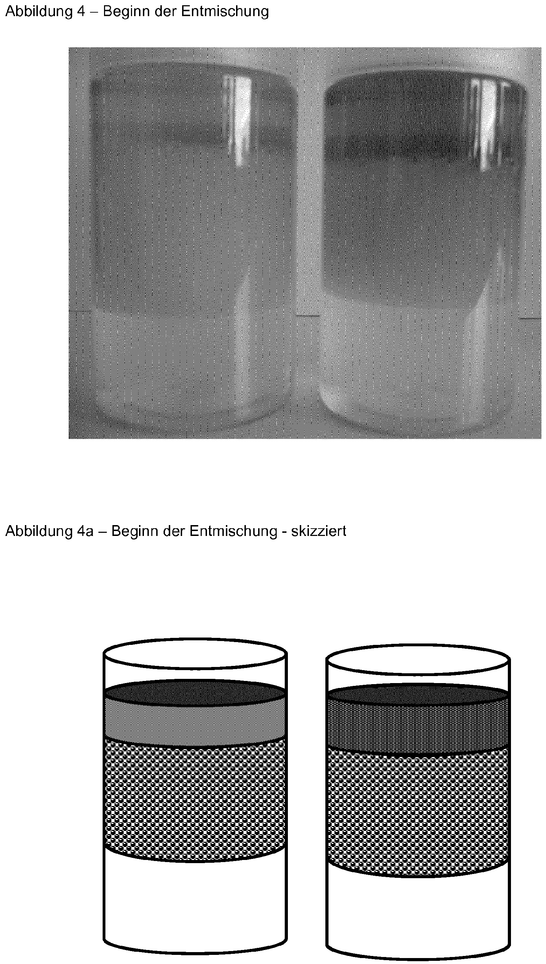

Benzethoniumchlorid kann daher in Zweiphasenzubereitungen zur Verlängerung des Phasentrennungsstartes nach dem Vermischen der beiden Phasen verwendet werden. Der Trennungsstart ist der Zeitpunkt ab dem die Trennung der beiden Phasen optisch erkennbar wird (s. Abbildung 4) und noch keine vollständig getrennten Phasen vorliegen.

Allein durch den Zusatz an Benzethoniumchlorid kann zudem die Trennungszeit verlängert werden, wie im Beispiel auf 15 Minuten.

Daher kann Benzethoniumchlorid in Zweiphasenzubereitungen zur Verlängerung der Phasentrennzeit nach dem Vermischen der beiden Phasen verwendet werden.

Als erfindungsgemäße Verlängerung (des Trennungsstartes bzw. der Phasentrennzeit) wird eine längere Zeitspanne als diejenige verstanden, die Zubereitungen ohne erfindungsgemäßes Benzethoniumchlorid aufweisen.

Diese Einflussnahme eines nur als Konservierungsmittel bekannten Stoffes war äußerst überraschend.

Durch den Zusatz an Benzethoniumchlorid zu zweiphasigen Zubereitungen wird die gewünschte Anwendung einer ausreichend langanhaltenden Mischung von Öl- und Wasserphase für den Konsumenten erst möglich.

Erfindungsgemäß kann der Anwender nun in ausreichend verlängerter Zeit das Zweiphasenprodukt anwenden ohne befürchten zu müssen, dass die Phasen schon wieder getrennt vorliegen.

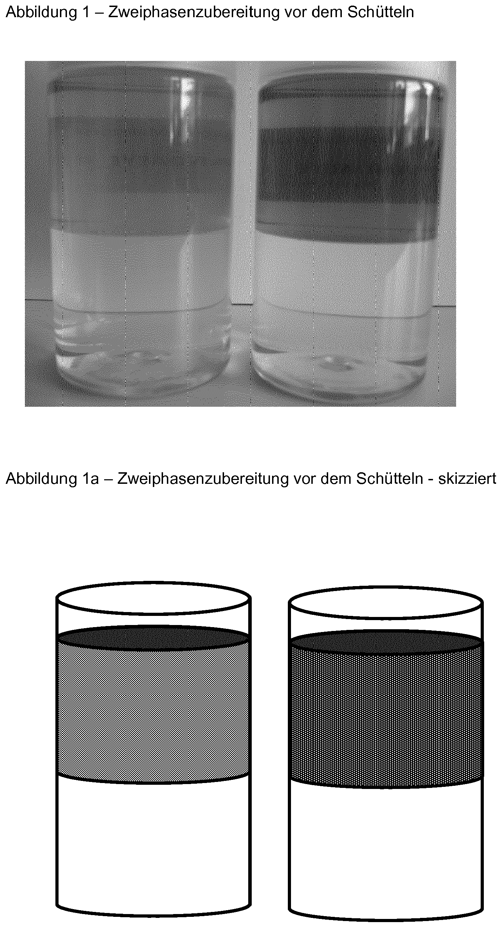

In den Abbildungen 1 bis 5 ist das Trennungsverhalten des erfindungsgemäßen Systems dargestellt.

Vor dem Schütteln zeigen die beiden erfindungsgemäßen Zweiphasenzubereitungen, wobei die Ölphase mit einem blauen Farbstoff versetzt ist, eine optisch klare Trennung der beiden Phasen (Abbildung 1).

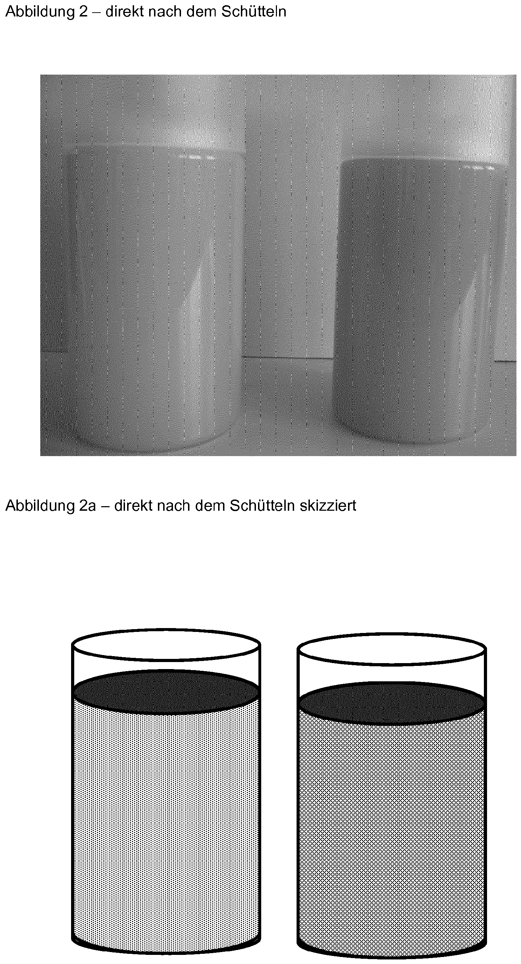

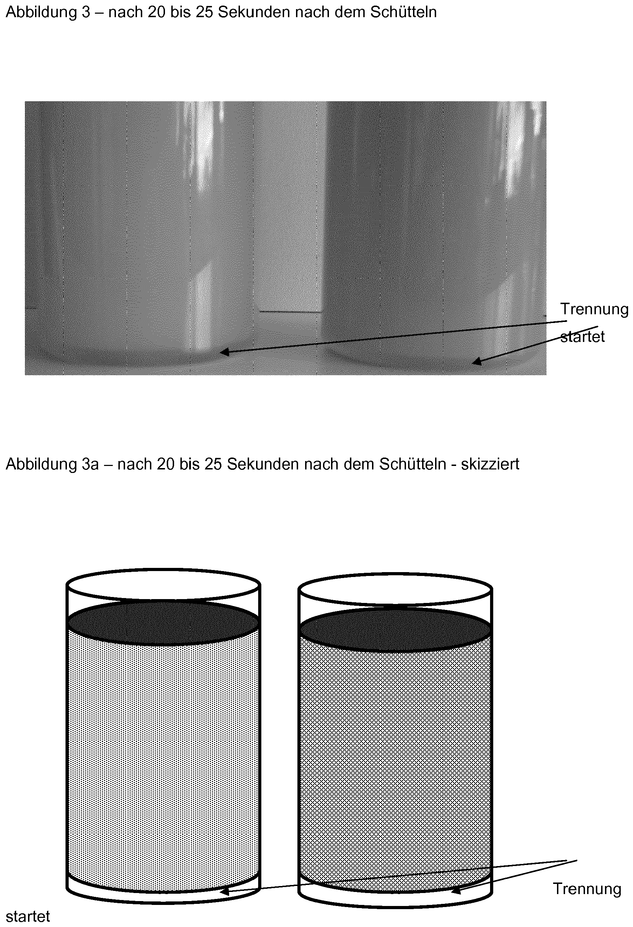

Beide Zubereitungen werden etwa 5- bis 10-mal geschüttelt, wie es der Normalverbraucher vor der Anwendung macht. Es zeigt sich eine Vermischung beider Phasen, die über 25 Sekunden anhält, wenn man nach dem Schütteln die Zubereitungen wieder in Ruhe lässt (Abbildungen 2 und 3).

Nach ca. 20-25 sec. erkennt man den Beginn der Entmischung (Abbildung 4).

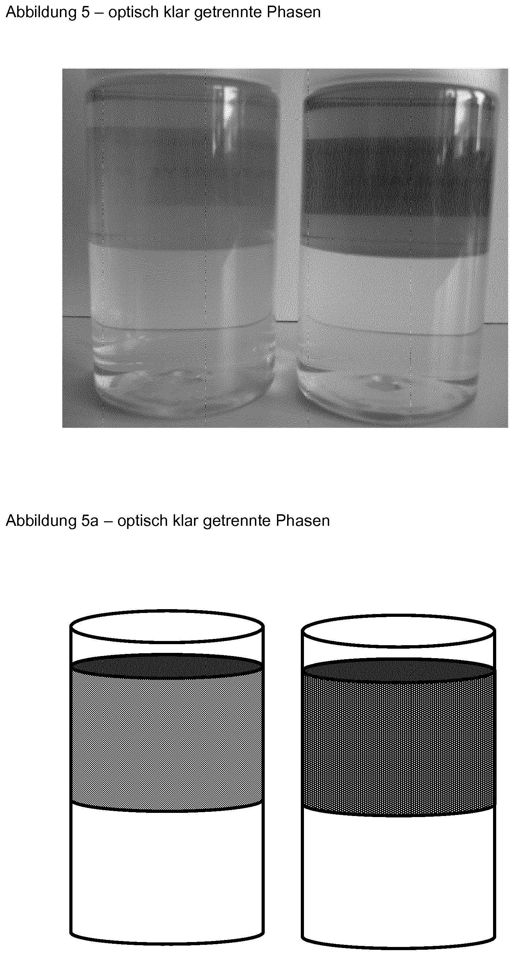

Die Trennung in zwei Phasen mit klarer Grenzfläche ist nach ca. 15 min ungefähr abgeschlossen, die Phasen sind dann jedoch noch leicht trübe, was auch immer davon abhängt wie stark und lange geschüttelt wurde (Abbildung 5). Nach <12 Stunden sind beide Phasen wieder klar.

Durch den alleinigen Zusatz an Benzethoniumchlorid wird die Grenzflächenspannung (GFS) stark herabgesetzt. Untersuchungen haben ergeben, dass Zubereitungen mit einer GFS kleiner 20 mN/m die erfindungsgemäßen Vorteile zeigen. Vorzugsweise sollte die GFS kleiner 10 mN/m, noch besser kleiner 4mN/m sein, um eine deutlich verlangsamte Trenngeschwindigkeit zu erhalten. Durch die Zugabe von beispielsweise 0,04 Gew.% Benzethonium Chlorides wird die GFS auf 3,5 mN/m gesenkt.

Die in den Beispielen (siehe Abbildungen 1 bis 5 oder Beispiel 508) dargestellte erfindungsgemäße Zubereitung zeigt eine GFS von 3,3 mN/m.

Ein weiteres Problem bei 2-phasigen Produkten zeigte sich bei Zusatz an nichtionischen Tensiden, da es durch die Mischung der beiden Phasen bei gleichzeitigem Vorhandensein eines nichtionischen Tensides zu optischen Phänomenen kommen kann, wie z.B. Schaumbildung an der Grenzfläche, emulgierte Phasen oder Phasenanteile, Trübungen einer oder beider Phasen.

Auch hier bestand die Herausforderung darin die 2-Phasen Produkte auch optisch für den Konsumenten attraktiv zu gestalten ohne Inkaufnahme obiger Nachteile.

Überraschend konnte durch Zugabe eines kurzkettigen Dimethicones (wie beispielsweise Belsil DM 10 von Wacker, ELEMENT 14 PDMS 20 von Momentive Performance Materials) die Zweiphasenzubereitung soweit optimiert werden, dass das Produkt nach einiger Zeit wieder in zwei klaren Phasen vorliegt, was für den Konsumenten sehr wichtig ist. BELSIL® DM 10 ist ein lineares, nicht-reaktives, unmodifiziertes Polydimethylsiloxan.

Als erfindungsgemäß kurzkettiges Dimethicon werden Dimethicone mit einer Viskosität im Bereich von 8 bis 30 cSt verstanden. Die kurzkettigen Dimethicone zeigen zudem eine mittlere Kettenlänge von 30 und weniger. Die kinematische Viskosität (Rotationsviskosität) wird bei 25°C gemäß DIN 53019 ermittelt.

**Tabelle 3: Dimethicone**

| Hersteller Momentive | Handelsname Elemtent 14 PDMS 20 | INCI Dimethicone | Viskosität 20 cSt | Gehalt in % 95,1; Hexadecamethylheptasiloxan | Gehalt in % 4,9; Octamethylcyclotetrasiloxan | PDMS Polydimethyl-siloxane; mittlere Kettenlänge 23,7 |
|---|---|---|---|---|---|---|
| Wacker | Belsil DM 10 | Dimethicone | 10 cSt | | 99,8; Tetradecamethyl-hexasiloxan | 10,3 |
| Dow Corning | Xiameter PMX-200 Silicone Fluid 100CS | Dimethicone | 100 cSt | | | 69-70 |

Bevorzugte Dimethicone sind die in der Tabelle 3 aufgeführten Dimethicone mit der allgemeinen Form [MDₙM] bzw. R₃SiO[R₂SiO]ₙSiR₃.

Das bevorzugte Dimethicon PDMS 20 besteht aus den in der Tabelle 3 genannten beiden Siloxanen Hexadecamethylheptasiloxan und Octamethylcyclotetrasiloxan und hat eine mittlere Kettenlänge von 23,7.

Das Dimethicone Belsil DM 10 besteht aus 99.8% Tetradecamethylhexasiloxan oder größeren Siloxaneinheiten und hat eine mittlere Kettenlänge von 10,3.

Nicht erfindungsgemäße, langkettige Dimethicone (wie z.B. Xiameter PMX-200 Silicone Fluid) haben eine Viskosität von 100 cSt und umfassen als Hauptsubstanz Polydimethylsiloxan (kurz PDMS) mit einer mittleren Kettenlänge von etwa 69-70.

Vorteilhaft wird der Anteil an Dimethiconen im Bereich von 5 bis 15 Gew.%, insbesondere 8 bis 12 Gew.%, bezogen auf die Gesamtmasse der Zubereitung, gewählt.

Weiterer Vorteil der kurzkettigen Dimethicone liegt in ihrer verbesserten Hautverträglichkeit und darin auf Cyclomethicone vollständig verzichten zu können.

Die erfindungsgemäßen Zubereitungen umfassen daher vorteilhaft weniger als 0,01 Gew.% Cyclomethicone und gelten so als Cyclomethiconfrei. Insbesondere liegt deren Anteil bei 0 Gew.%, bezogen auf die Gesamtmasse der Zubereitung.

Desweiteren wurde beobachtet, dass in der erfindungsgemäßen Zubereitung die Zugabe von Benzethoniumchlorid und etwa 0,04 Gew.% eines nichtionischen Tensids eine klarere Grenzfläche erzeugt. Es kommt dabei zu keiner Schaumbildung.

Vorteilhaft umfasst die Zubereitung ein oder mehrere nichtionische Tenside, insbesondere Caprylyl/Capryl Glucosid.

Der Anteil an nichtionischen Tensiden, insbesondere Caprylyl/Capryl Glucosid, liegt bevorzugt im Bereich von 0,01 bis 2 Gew.%, insbesondere im Bereich um 0,04 Gew.%, bezogen auf die Gesamtmasse der Zubereitung.

Die erfindungsgemäße zweiphasige Zubereitung verzichtet auf weitere Emulgatoren oder Tenside. D.h. erfindungsgemäße Zubereitungen umfassen außer ggf. den nichtionischen Tensiden, insbesondere Caprylyl/Capryl Glucosid, weniger als 0,01 Gew.% weitere Tenside oder Emulgatoren.

Ein weiterer bemerkenswerter Faktor bei 2-phasigen Produkten ist deren Abfüllverhältnis. Es stellte sich heraus die richtig Balance von Wasser- und Ölphase zu finden, damit eine optimale Reinigungswirkung erzeugt werden kann. Zu wenig Öl wirkt sich nachteilig auf die Reinigungsleistung aus.

Idealerweise wird das Gewichtsverhältnis der Wasser- zur Ölphase im Bereich von 4 zu 1 bis 2 zu 3, insbesondere 7 zu 3 bis 1 zu 1, vorteilhaft 1 zu 1, gewählt.

Da Zweiphasenzubereitungen häufig als Reinigungszubereitungen und insbesondere als Augen-Make-up Entferner verwendet werden, ist eine sehr gute Verträglichkeit vor allem im Bereich um und am Auge wichtig.

Durch den erfindungsgemäßen Einsatz von Benzethoniumchlorid konnte eine sehr gute Haut- und vor allem Augenverträglichkeit erreicht werden. Dies konnte insbesondere in Kombination mit einer geringen Menge an Phenoxyethanol und dem besonders gut verträglichen nichtionischen Tensid Caprylyl/Capryl Glucosid erzielt werden. Durch die Zugabe eines kurzkettigen Dimethicons (Elemtent 14 PDMS 20) konnte die Verträglichkeit noch einmal gesteigert werden.

In einem Anwendungstest mit 68 Probandinnen (Anwendungszeitraum pro Produkt 5d) konnte die gute Verträglichkeit im Vergleich zu einem Marktprodukt mit D5 (Cyclomethicon) deutlich gesteigert werden.

**Tabelle 4: Zweiphasenprodukte - Vergleichsuntersuchungen Hautverträglichkeit**

| INCI (EU) | N° 507 Marktformel mit D5 und Konservierung mit Methylisothiazolinon | N° 508 Incl. kurzkettiges Dimethicon | N° 528 silikonfrei |
|---|---|---|---|
| Isopropyl Palmitate | 9,9000 | 10,0000 | 19,5000 |
| Isododecane | 30,2000 | 30,5000 | 30,5000 |
| Cyclomethicone | 9,9000 | | |
| Cl 60725 | 0,0005 | 0,0005 | 0,0005 |
| Dimethicone (ELEMENT 14 PDMS 20) | | 9,5000 | |
| Methylisothiazolinone | 0,0400 | | |
| Phenoxyethanol | 0,6000 | 0,3000 | 0,3000 |
| Benzethonium Chloride | | 0,0400 | 0,0400 |
| Sodium Chloride | 0,5000 | 0,2000 | 0,2000 |
| Aqua | Qsp 100% | Qsp 100% | Qsp 100% |
| Aqua + Trisodium EDTA | 1,0000 | 0,5000 | 0,5000 |
| Aqua + Caprylyl/Capryl Glucoside | | 0,0400 | 0,0400 |

Auf einer Skala von 1-7 (wobei 1 = trifft nicht zu und 7 = trifft zu bedeutet, wurden die Produkte miteinander verglichen.

**Tabelle 5: Anwendungstest - Haut- und Augenverträglichkeit**

| Frage | 508 | 528 | 507 |
|---|---|---|---|
| Das Produkt reinigt auf sanfte Weise | 5,8 | 5,8 | 5,6 |
| Das Produkt ist besonders mild zur empfindlichen Augenpartie | 5,9 | 5,7 | 5,6 |
| Das Produkt reizt die Augenpartie nicht | 6,1 | 5,9 | 5,6 |
| Das Produkt brennt nicht am Auge | 6,1 | 5,9 | 5,8 |
| Das Produkt hinterlässt ein angenehmes Gefühl auf der Haut | 5,5 | 5,4 | 5 |
| Das Produkt hinterlässt keinen öligen Rückstand am Auge | 5,5 | 5,4 | 4,9 |
| Das Produkt hinterlässt keinen unangenehmen Film im Auge | 5,7 | 5,6 | 5,1 |
| | | | |

| Verträglichkeit im Augenbereich | | | |
|---|---|---|---|
| sehr gut | 73% | 64% | 59% |
| gut | 27% | 36% | 41% |
| weniger gut | 0 | 0 | 0 |
| nicht gut | 0 | 0 | 0 |

| | | | |
|---|---|---|---|
| ≥ 6,0 - sehr gutes Niveau 5,0 bis 5,9 - gutes Niveau 4,0 bis 4,9 - moderates Niveau % - Prozent der Probanden | | | |

Auszug aus dem finalen Report des untersuchenden Institutes:
Darüber hinaus setzt sich 508 hinsichtlich weiterer Eigenschaften signifikant vom Marktprodukt 507 ab:
   - das Produkt reinigt auf sanfte Weise
   - das Produkt ist besonders mild zur empfindlichen Augenpartie
   - das Produkt reizt die Augenpartie nicht
   - das Produkt brennt nicht am Auge
   - das Produkt hinterlässt keinen unangenehmen Film im Auge.
   - Die Verträglichkeit im Augenbereich wird für 508 im Vergleich zu 528 und 507 etwas häufiger als sehr gut bewertet.

Bekannte Produkte setzen als Konservierungsmittel Polyaminopropyl Biguanide ein. Dieses Konservierungsmittel wird jedoch nicht als unbedenklich angesehen.

Vorteilhaft umfassen die erfindungsgemäßen Zubereitungen weniger als 0,01 Gew.%, insbesondere kein Polyaminopropyl Biguanid.

Die erfindungsgemäße Zubereitung umfasst vorteilhaft neben Wasser und Benzethoniumchlorid Isododecane, kurzkettiges Dimethicon und Phenoxyethanol.

Durch die Zugabe von Phenoxyethanol zu der erfindungsgemäßen Zubereitung konnte die Bildung von sogenannten "Fettaugen" weiter minimiert bzw. verhindert werden. Fettaugen sind optisch sichtbare Tropfen, die sich im Bereich der Wasserphase oder auch im gesamten Packmittel an der Packmittelwandung anlagern können.

Die kosmetischen oder dermatologischen Zubereitungen gemäß der Erfindung können ferner kosmetische Hilfsstoffe und weitere Wirkstoffe enthalten, wie sie üblicherweise in solchen Zubereitungen verwendet werden, z. B. Substanzen zum Verhindern des Schäumens, Farbstoffe und Farbpigmente, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate, Selbstbräuner, Puffer, pH Regulatoren, Pflanzliche Extrakte, Puder, Sebumabsorbierende Substanzen, UV-Filter, Wirkstoffe wie zum Beispiel Anti Age, Anti-Cellulite, Anti Akne, Anti-Rosacea, Anti-Neurodermitis, Antioxidantien, Moisturiser, Chelatbildner, Antitraspirantien, Bleich und Färbemittel etc. sofern der Zusatz die geforderten Eigenschaften hinsichtlich der Phasentrennung und Hautverträglichkeit nicht behindert.

Vorteilhaft umfassend die erfindungsgemäßen Zubereitungen Natriumchlorid und Trisodium-Ethylenediaminetetraacetic acid sowie gegebenenfalls ein oder mehrere Wirkstoffe, Parfume, Farbstoffe und/oder Hautbefeuchtungsmittel.

Bevorzugte Hautbefeuchtungsmittel sind Glycerin und/oder Glyceryl Glucosid. Vorteilhaft wird Glycerin und/oder Glyceryl Glucosid der Zubereitung zu einem Anteil bis zu jeweils 5 Gew.% zugesetzt, vorzugsweise 1 bis 2%, besonders bevorzugt 1 Gew.%, bezogen auf die Gesamtmasse der Zubereitung.

Vorteilhaft umfasst die Zubereitung ein oder mehrere Farbstoffe, die entweder die Wasser- oder die Ölphase anfärben. Man kommt somit zu einer optisch attraktiven Ausführungsform und die Phasen sind unterscheidbar gestaltet.

Als öllösliche Farbstoffe werden insbesondere Cl 61565 (1,4-bis(p-tolylamino)anthraquinone) und Cl 60725 (1-Hydroxy-4-(p-toluidino)anthrachinon) sowie als wasserlöslicher Farbstoff insbesondere Cl 16035 (FD&C Red 40; 6-Hydroxy-5-((6-methoxy-4-sulfo-m-tolyl)azo)-2-naphthalenesulfonic acid disodium salt) gewählt.

Erfindungsgemäße Ausführungsformen können obige Bestandteile umfassen, es kann jedoch auch auf einzelne Bestandteile bevorzugt verzichtet werden.

Vorteilhaft umfassen die erfindungsgemäßen Zubereitungen weniger als 0,01 Gew.%, insbesondere 0 Gew.% an Polymeren und/oder Verdickern, jeweils bezogen auf die Gesamtmasse der Zubereitung.

Vorteilhaft umfasst die erfindungsgemäße Zubereitung neben Benzethoniumchlorid und gegebenenfalls ein oder mehreren nichtionischen Tensiden keine weiteren Emulgatoren und/oder Tenside.

Vorteilhaft umfasst die erfindungsgemäße 2-phasige Zubereitung neben Benzethoniumchlorid, insbesondere zu einem Anteil von 0,01 bis 1 Gew.%, ein oder mehrere Dimethicone mit einer Viskosität im Bereich von 8 bis 30 cSt (25°C), ein oder mehrere nichtionische Tenside, insbesondere Caprylyl/Capryl Glucosid, Isododecan und Phenoxyethanol und es wird auf Cyclomethicone, Polyaminopropyl Biguanid, Polymere, Verdicker und weitere Tenside und Emulgatoren verzichtet.

Zur Herstellung der erfindungsgemäßen Zweiphasenzubereitungen kann vorteilhaft wie nachfolgend beispielhaft dargestellt vorgegangen werden.

Bei der Herstellung der Wasserphase ist eine bestimmte Herstellanweisung einflussreich, damit es nicht zu unerwünschten Ausfällungen bzw. Trübungen kommt. Die Zugabe sollte in folgender Reihenfolge erfolgen:

| | | |
|---|---|---|
| 1. | Aqua | Rohstoffe mischen, bis klar/komplett gelöst! |
| 2. | Phenoxyethanol | |
| 3. | Benzethonium Chloride | |
| 4. | Trisodium EDTA | Zugeben und rühren |
| 5. | Sodium Chloride | Zugeben und rühren |
| 6. | Caprylyl/Capryl Glucoside | Zum Schluss zugeben und rühren! |

Vorteilhaft ist auch folgende Herstellung:

| | | |
|---|---|---|
| 1. | Phenoxyethanol | Rohstoffe mischen, bis klar/komplett gelöst! |
| 2. | Benzethonium Chloride | |
| 3. | Aqua | |
| 4. | Trisodium EDTA | Zugeben und rühren |
| 5. | Sodium Chloride | Zugeben und rühren |
| 6. | Caprylyl/Capryl Glucoside | Zum Schluss zugeben und rühren! |

oder

| | | |
|---|---|---|
| 1. | Benzethonium Chloride | Rohstoffe mischen, bis klar/komplett gelöst! |
| 2. | Aqua | |
| 3. | Phenoxyethanol | |
| 4. | Trisodium EDTA | Zugeben und rühren |
| 5. | Sodium Chloride | Zugeben und rühren |
| 6. | Caprylyl/Capryl Glucoside | Zum Schluss zugeben und rühren! |

oder

| | | |
|---|---|---|
| 1. | Aqua | Rohstoffe mischen, bis klar/komplett gelöst! |
| 2. | Glyceryl Glucoside | |
| 3. | Glycerin | |
| 4. | Phenoxyethanol | |
| 5. | Benzethonium Chloride | |
| 6. | Trisodium EDTA | Zugeben und rühren |
| 7. | Sodium Chloride | Zugeben und rühren |
| 8. | Caprylyl/Capryl Glucoside | Zum Schluss zugeben und rühren! |

Nachfolgende Beispielrezepturen sollen die vorliegende Erfindung erläutern. Zahlenangaben beziehen sich auf Gewichtsanteile bezogen auf die Gesamtmasse der Zubereitung.

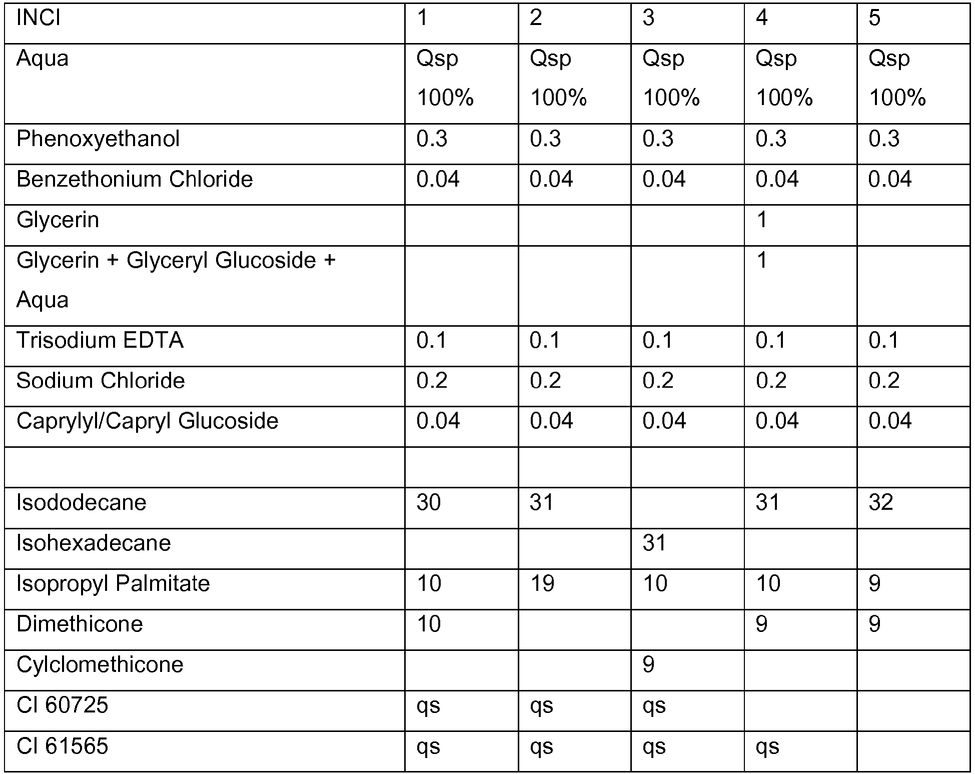

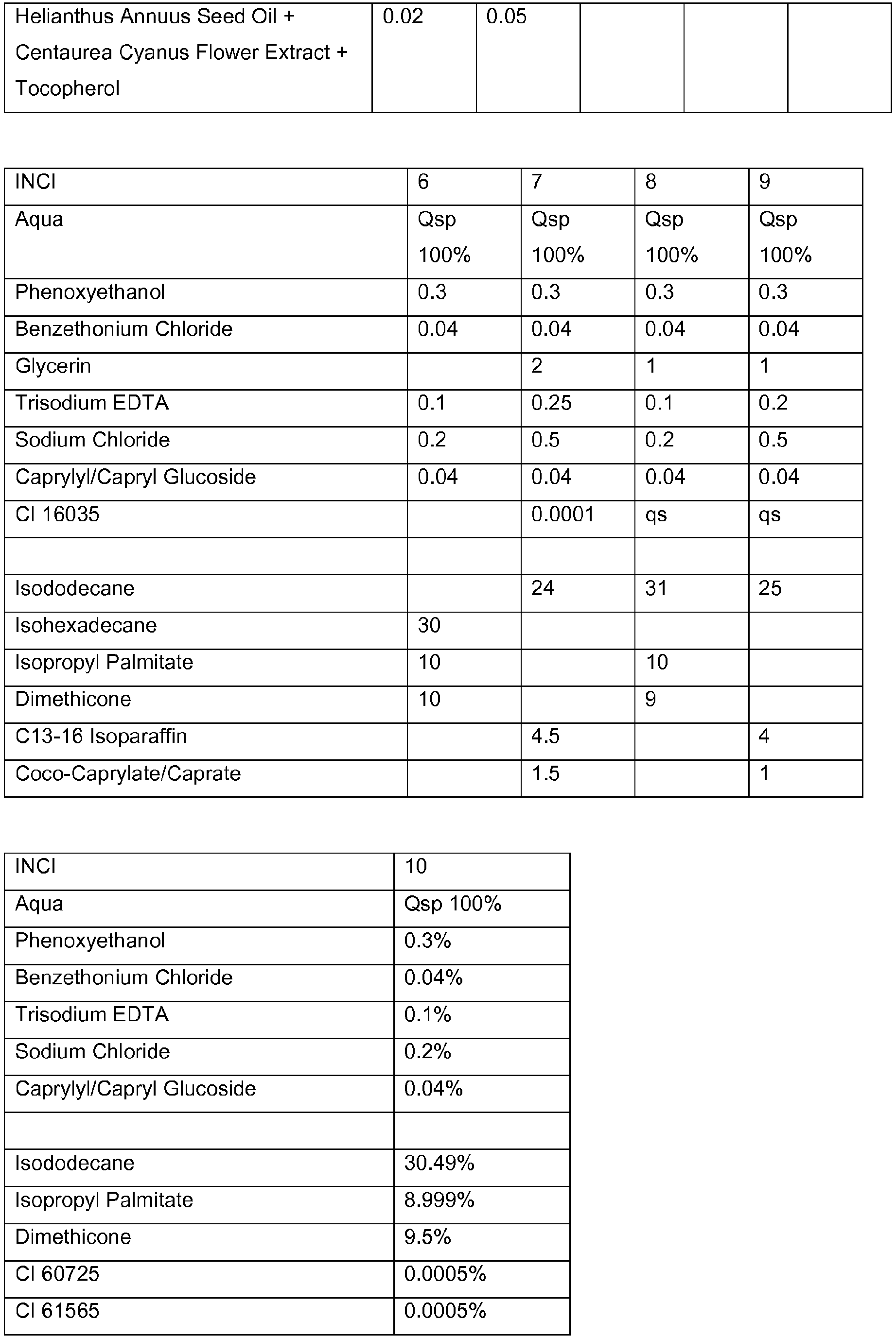

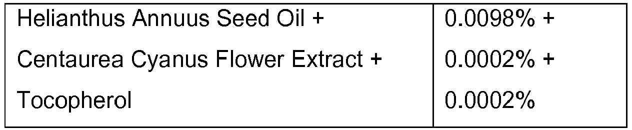

## Patentansprüche

1. Kosmetisches oder dermatologisches horizontal getrenntes 2-phasiges System umfassend eine Öl- und eine Wasserphase und Benzethoniumchlorid.

2. System nach Anspruch 1 **dadurch gekennzeichnet, dass** der Anteil an Benzethoniumchlorid im Bereich von 0,01 bis 1 Gew.%, insbesondere 0,03 bis 0,05 Gew.%, bezogen auf die Gesamtmasse der Zubereitung, gewählt wird.

3. System nach einem der vorstehenden Ansprüche **dadurch gekennzeichnet, dass** die Ölphase gebildet wird aus einem oder mehreren Bestandteilen gewählt aus Isododecane, Isohexadecane, Isopropylpalmitat, C13-16 Isoparaffin, Coco-Caprylat/Caprat und Dimethicon oder die Ölphase umfasst ein oder mehrere dieser Bestandteile.

4. System nach Anspruch 3 umfassend ein oder mehrere Dimethicone mit einer Viskosität im Bereich von 8 bis 30 cSt (25°C).

5. System nach Anspruch 3 oder 4 **dadurch gekennzeichnet, dass** der Anteil an Dimethiconen im Bereich von 5 bis 15 Gew.%, insbesondere 8 bis 12 Gew.%, bezogen auf die Gesamtmasse der Zubereitung, gewählt wird.

6. System nach einem der vorstehenden Ansprüche umfassend ein oder mehrere nichtionische Tenside.

7. System nach Anspruch 6 umfassend Caprylyl/Capryl Glucosid.

8. System nach Anspruch 6 oder 7 **dadurch gekennzeichnet, dass** der Anteil an nichtionischen Tensiden im Bereich von 0,01 bis 2 Gew.%, insbesondere im Bereich um 0,04 Gew.%, bezogen auf die Gesamtmasse der Zubereitung, gewählt wird.

9. System nach einem der vorstehenden Ansprüche umfassend neben Benzethoniumchlorid und gegebenenfalls ein oder mehreren nichtionischen Tensiden keine weiteren Emulgatoren und/oder Tenside.

10. System nach einem der vorstehenden Ansprüche umfassend Phenoxyethanol.

11. System nach einem der vorstehenden Ansprüche umfassend neben Wasser und Benzethoniumchlorid Isododecan, Dimethicon und Phenoxyethanol.

12. System nach einem der vorstehenden Ansprüche umfassend Natriumchlorid und Trisodium- Ethylenediaminetetraacetic acid.

13. System nach einem der vorstehenden Ansprüche umfassend ein oder mehrere Wirkstoffe, Parfume, Farbstoffe und/oder Hautbefeuchtungsmittel.

14. System nach einem der vorstehenden Ansprüche **dadurch gekennzeichnet, dass** die Zubereitung neben den gegebenenfalls nicht-ionischen Tensiden weniger als 0,01 Gew.%, insbesondere 0 Gew.%, jeweils an Cyclomethiconen, Polyaminopropyl Biguanid, Polymeren, Verdickern, Tenside und/oder Emulgatoren umfasst, jeweils bezogen auf die Gesamtmasse der Zubereitung.

15. System nach einem der vorstehenden Ansprüche **dadurch gekennzeichnet, dass** das Gewichtsverhältnis der Wasser- zur Ölphase im Bereich von 4 zu 1 bis 2 zu 3, insbesondere 7 zu 3 bis 1 zu 1, vorteilhaft 1 zu 1, gewählt wird.

16. Verwendung des 2-phasigen Systems nach einem der vorstehenden Ansprüche als Make-up Entferner, insbesondere im Bereich um die Augen.

17. Verwendung von Benzethoniumchlorid in einem horizontal getrennten 2-phasigen System zur Verlängerung des Phasentrennungsstartes nach dem Vermischen der beiden Phasen.

18. Verwendung von Benzethoniumchlorid in einem horizontal getrennten 2-phasigen System zur Verlängerung der Phasentrennzeit nach dem Vermischen der beiden Phasen.

## Claims

1. Cosmetic or dermatological horizontally separated 2-phase system comprising an oil phase and a water phase and benzethonium chloride.

2. System according to Claim 1, **characterized in that** the proportion of benzethonium chloride is selected in the range of 0.01 to 1% by weight, in particular 0.03 to 0.05% by weight, based on the total mass of the preparation.

3. System according to either of the preceding claims, **characterized in that** the oil phase is formed from one or more constituents selected from isododecane, isohexadecane, isopropyl palmitate, C13-16 isoparaffin, coco-caprylate/caprate and dimethicone or the oil phase comprises one or more of these constituents.

4. System according to Claim 3 comprising one or more dimethicones having a viscosity in the range of 8 to 30 cSt (25°C).

5. System according to Claim 3 or 4, **characterized in that** the proportion of dimethicones is selected in the range of 5 to 15 % by weight, especially 8 to 12 % by weight, based on the total mass of the preparation.

6. System according to any of the preceding claims, comprising one or more non-ionic surfactants.

7. System according to Claim 6 comprising caprylyl/capryl glucoside.

8. System according to Claim 6 or 7, **characterized in that** the proportion of non-ionic surfactants is selected in the range of 0.01 to 2% by weight, especially in the range around 0.04% by weight, based on the total mass of the preparation.

9. System according to any of the preceding claims comprising, besides benzethonium chloride and optionally one or more non-ionic surfactants, no further emulsifiers and/or surfactants.

10. System according to any of the preceding claims comprising phenoxyethanol.

11. System according to any of the preceding claims comprising, in addition to water and benzethonium chloride, isododecane, dimethicone and phenoxyethanol.

12. System according to any of the preceding claims comprising sodium chloride and trisodium ethylenediaminetetraacetic acid.

13. System according to any of the preceding claims, comprising one or more active ingredients, perfumes, colourants and/or skin moisturisers.

14. System according to any of the preceding claims, **characterized in that** the preparation, in addition to the optional non-ionic surfactants, comprises less than 0.01% by weight, especially 0% by weight each of cyclomethicones, polyaminopropyl biguanide, polymers, thickeners, surfactants and/or emulsifiers, based in each case on the total mass of the preparation.

15. System according to any of the preceding claims, **characterized in that** the ratio by weight of the water phase to the oil phase is selected in the range of 4:1 to 2:3, particularly 7:3 to 1:1, advantageously 1:1.

16. Use of the 2-phase system according to any of the preceding claims as make up remover, especially in the area around the eyes.

17. Use of benzethonium chloride in a horizontally separated 2-phase system for prolonging the start of the phase separation after mixing the two phases.

18. Use of benzethonium chloride in a horizontally separated 2-phase system for prolonging the phase separation time after mixing the two phases.

## Revendications

1. Système cosmétique ou dermatologique biphasé séparé horizontalement, comprenant une phase huileuse et une phase aqueuse et du chlorure de benzéthonium.

2. Système selon la revendication 1, **caractérisé en ce que** la proportion de chlorure de benzéthonium est choisie dans la plage allant de 0,01 à 1 % en poids, notamment de 0,03 à 0,05 % en poids, par rapport à la masse totale de la préparation.

3. Système selon l'une quelconque des revendications précédentes, **caractérisé en ce que** la phase huileuse est formée à partir d'un ou de plusieurs constituants choisis parmi les isododécanes, les isohexadécanes, le palmitate d'isopropyle, une isoparaffine en C13-16, le caprylate/caprate de coco et la diméthicone, ou la phase huileuse comprend un ou plusieurs de ces constituants.

4. Système selon la revendication 3, comprenant une ou plusieurs diméthicones ayant une viscosité dans la plage allant de 8 à 30 cSt (25 °C).

5. Système selon la revendication 3 ou 4, **caractérisé en ce que** la proportion de diméthicones est choisie dans la plage allant de 5 à 15 % en poids, notamment de 8 à 12 % en poids, par rapport à la masse totale de la préparation.

6. Système selon l'une quelconque des revendications précédentes, comprenant un ou plusieurs tensioactifs non ioniques.

7. Système selon la revendication 6, comprenant du glucoside de caprylyle/capryle.

8. Système selon la revendication 6 ou 7, **caractérisé en ce que** la proportion de tensioactifs non ioniques est choisie dans la plage allant de 0,01 à 2 % en poids, notamment d'environ 0,04 % en poids, par rapport à la masse totale de la préparation.

9. Système selon l'une quelconque des revendications précédentes, ne comprenant pas d'autres émulsifiants et/ou tensioactifs en plus du chlorure de benzéthonium et éventuellement d'un ou de plusieurs tensioactifs non ioniques.

10. Système selon l'une quelconque des revendications précédentes, comprenant du phénoxyéthanol.

11. Système selon l'une quelconque des revendications précédentes, comprenant de l'isododécane, de la diméthicone et du phénoxyéthanol en plus de l'eau et du chlorure de benzéthonium.

12. Système selon l'une quelconque des revendications précédentes, comprenant du chlorure de sodium et de l'acide éthylène-diamine-tétraacétique trisomique.

13. Système selon l'une quelconque des revendications précédentes, comprenant un ou plusieurs agents actifs, parfums, colorants et/ou agents d'hydratation de la peau.

14. Système selon l'une quelconque des revendications précédentes, **caractérisé en ce que** la préparation comprend en plus des tensioactifs éventuellement non ioniques moins de 0,01 % en poids, notamment 0 % en poids, respectivement de cyclométhicones, de polyaminopropyl-biguanide, de polymères, d'épaississants, de tensioactifs et/ou d'émulsifiants, à chaque fois par rapport à la masse totale de la préparation.

15. Système selon l'une quelconque des revendications précédentes, **caractérisé en ce que** le rapport en poids entre la phase aqueuse et la phase huileuse est choisi dans la plage allant de 4 sur 1 à 2 sur 3, notamment de 7 sur 3 à 1 sur 1, avantageusement de 1 sur 1.

16. Utilisation du système biphasé selon l'une quelconque des revendications précédentes en tant que produit démaquillant, notamment dans la zone autour des yeux.

17. Utilisation de chlorure de benzéthonium dans un système biphasé séparé horizontalement pour prolonger le début de la séparation des phases après le mélange des deux phases.

18. Utilisation de chlorure de benzéthonium dans un système biphasé séparé horizontalement pour prolonger le temps de séparation des phases après le mélange des deux phases.
